# EUROPEAN PATENT APPLICATION

(11) **EP 2 003 442 A2**
(43) Date of publication of application: **17.12.2008**
(21) Application number: 07736970.0
(22) Date of filing: 28.03.2007
(51) Int. Cl.: G01N 21/64

(54) **METHOD OF EVALUATING CELL FUNCTION, SYSTEM FOR EVALUATING CELL FUNCTION, FLUORESCENT MICROSCOPE SYSTEM, PHOTOTHERAPY METHOD AND PHOTOTHERAPY SYSTEM**

(30) Priority: 31.03.2006 JP 2006098712
(71) Applicant: Nikon Corporation, Tokyo 100-8331 (JP); SAKURAI, Takashi, Hamamatsu-shi, Shizuoka 433-8121 (JP)
(72) Inventor: SAKURAI, Takashi, Hamamatsu-shi Shizuoka 433-8121 (JP); IKI, Yoichi, Tokyo 100-8331 (JP)
(74) Representative: Petzold, Silke
(86) International application number: PCT/JP2007/000313
(87) International publication number: WO 2007/116582

(57) **Abstract**

A proposition of the present invention is to accurately evaluate a phototoxic property (cell function) concerning a living cell. To this end, an evaluating method of cell function of the present invention includes the operations of dyeing a specific site (41) of the living cell (40) with a fluorescent dye, irradiating the living cell (40) with light to measure changes in brightness of resulting fluorescence generated at an adjacent site (43) of the specific site (41), and evaluating the phototoxic property based on the brightness changes. In the event of functional depression in the specific site (41), the fluorescent dye cannot be retained therein and is extravasated into the adjacent site (43) through the membrane of the specific site. The present invention can measure the extent of this extravasation, making it possible to accurately evaluate the phototoxic property.

## Description

### TECHNICAL FIELD

The present invention relates to an evaluating method of cell function, an evaluating system of cell function, a fluorescent microscope system, a photodynamic therapy method, and a photodynamic therapy system, applicable to photochemistry, photophysiology, photodynamic effects, and the like in life science.

### BACKGROUND ART

Chromophore-assisted laser inactivation (CALI) and phot dynamic therapy (PDT) are among the techniques of photodynamic therapy. The former is the technique that suppresses activity of molecules, and the latter is the technique that induces damage to the cell membrane, organelles, and DNA to cause cell death. The letter technique, PDT, is effective in killing cancer cells.
PDT works under the principle that a fluorescent dye in cells absorbs light and creates singlet oxygen and other chemical species, which are generated as by-products when the dye releases fluorescence. These chemical species would then damage part of cells to induce functional depression or cell death (see Patent Document 1, Non-Patent Document 1 , for example)

In photodynamic therapy, in order to find therapeutic effects or side effects, it is important to accurately evaluate the damage (functional depression) incurred to the living cells by irradiation of light. In this specification, the term "phototoxic property" is used to describe the extent of functional depression incurred to the living cells by irradiation of light.
Patent Document 1: Japanese Unexamined Patent Application Publication No. 2001-4542
Non-Patent Document 1: Takato YOSHIDA, Eiji KAWANO, Takashi SAKURAI, kisojikken moderu kara rinshou deno PDT monitaringu no kanousei wo saguru, Nihon Laser Chiryou Gakkaishi, Vol. 2, No. 2, pp. 67-71, published on January, 2004

### DISCLOSURE OF THE INVENTION

### PROBLEMS TO BE SOL VED BY THE INVENTION

Conventionally, phototoxic property has been evaluated only broadly based on whether cells have been killed by irradiation of light. In some techniques, the phototoxic property during light exposure is evaluated in real time by measuring the rate or extent of fluorescence bleaching. However, this method suffers from inaccuracy because the bleaching (decay of fluorescence brightness) is not well correlated with phototoxic property (functional depression of living cells).
Accordingly, it is a proposition of the present invention to provide an evaluating method of cell function, an evaluating system of cell function, and a fluorescent microscope system that are capable of accurately evaluating phototoxic property. Another proposition of the present invention is to provide a photodynamic therapy method capable of realizing an appropriate therapy, and a photodynamic therapy system suitable for such a photodynamic therapy method.

### MEANS FOR SOLVING THE PROBLEMS

An evaluating method of cell function of the present invention is a method of evaluating a cell function concerning a living cell, and the method includes dyeing operation of dyeing a specific site of the living cell with a fluorescent dye, measuring operation of measuring a brightness value of fluorescence generated at an adjacent site of the specific site as a result of irradiation of the stained living cell with light, and evaluating operation of evaluating the cell function based on changes in the measured brightness value.

The fluorescent dye may be Rhodamin 123.
Further, an evaluating method of cell function of the present invention may further include the operation of controlling the irradiation of light to the stained living cell at a predetermined timing according to the evaluation of cell function.
Further, in the evaluating operation, the cell function may be evaluated based on a peak of a curve representing the brightness changes.

Further, in the evaluating operation, the cell function may be evaluated based on an amount of light irradiation being spent until the brightness change curve reaches the peak.
Further, in the evaluating operation, the cell function may be evaluated based on a brightness value at the peak of the brightness change curve.
Further, the adjacent site of the specific site may be an area specified by a rectangular shaped frame, or an area specified by a closed and free curved frame or a closed and multiangular shaped frame in which the specific site is excluded from an area including the specific site and an area adjacent to the specific site.

Further, the brightness value may be a maximum brightness value or a mean brightness value of a plurality of fluorescence brightness values measured in the adjacent site.
Further, the specific site may be a mitochondrion, and the adjacent site may be a cellular cytoplasm.

An evaluating system of cell function of the present invention is a system that evaluates a cell function concerning a living cell, the living cell including a specific site stained with a fluorescent dye in advance, and the evaluating system of cell function includes an irradiating unit that irradiates the stained living cell with light, a measuring unit that measures a brightness value of fluorescence generated at an adjacent site of the specific site as a result of the irradiation of light, and an evaluating unit that evaluates the cell function based on changes in the measured brightness value.

The fluorescent dye may be Rhodamin 123.
Further, an evaluating system of cell function of the present invention may further include a controlling unit that controls the irradiation of light to the stained living cell at a predetermined timing according to the evaluation of cell function.
Further, the evaluating unit may evaluate the cell function based on a peak of a curve representing the brightness changes.

Further, the evaluating unit may evaluate the cell function based on an amount of light irradiation being spent until the brightness change curve reaches the peak.
Further the evaluating unit may evaluate the cell function based on a brightness value at the peak of the brightness change curve.
Further, the adjacent site of the specific site may be an area specified by a rectangular shaped frame, or an area specified by a closed and free curved frame or a closed and multangular shaped frame in which the specific site is excluded from an area including the specific site and an area adjacent to the specific site.

Further, the brightness value may be a maximum brightness value or a mean brightness value of a plurality of fluorescence brightness values measured in the adjacent site.
A fluorescent microscope system of the present invention includes an excitation unit that irradiates a living cell with excitation light, an observing unit that acquires a fluorescence image of the living cell, and an evaluating system of cell function of the present invention, in which the excitation unit and the observing unit are also used as the irradiating unit and the measuring unit, respectively, of the evaluating system.

Further, a photodynamic therapy method of the present invention is a method that irradiates a living cell with light, the method including dyeing operation of dyeing a specific site of the living cell with a fluorescent dye, therapeutic operation of irradiating the stained living cell with light, and evaluating operation of evaluating a cell function concerning the living cell by an evaluating method of cell function of the present invention.
Further, a photodynamic therapy system of the present invention is a system that irradiates a living cell with light, the living cell including a specific site stained with a fluorescent dye in advance, the system including a therapeutic unit that irradiates the stained living cell with light, a measuring unit that measures a brightness value of fluorescence generated at an adjacent site of the specific site as a result of the irradiation of light, and a presenting unit that presents to an operator changes in the measured brightness value, in real time.

The fluorescent dye may be Rhodamin 123.
Further, the adjacent site of the specific site may be an area specified by a rectangular shaped frame, or an area specified by a closed and free curved frame or a closed and multiangular shaped frame in which the specific site is excluded from an area including the specific site and an area adjacent to the specific site.
Further, the brightness value may be a maximum brightness value or a mean brightness value of a plurality of fluorescence brightness values measured in the adjacent site.

### ADVANTAGE OF THE INVENTION

The present invention realizes an evaluating method of cell function, an evaluating system of cell function, and a fluorescent microscope system that are capable of accurately evaluating phototoxic property. The invention also realizes a photodynamic therapy method capable of realizing an appropriate therapy, and a photodynamic therapy system suitable for such a photodynamic therapy method.

### BRIEF DESCRIPTION OF THE DRAWINGS

Fig. 1 is a diagram showing a configuration of a system of a First Embodiment.
Fig. 2 is a diagram explaining fluorescence images I₁, I₂, ..., I_{N}.
Fig. 3 is a flow chart representing an operation of a CPU 22 evaluating a phototoxic property.
Fig. 4 is a diagram explaining step S1.
Fig. 5 is a diagram explaining steps S2 and S3.
Fig. 6 is a diagram showing changes in brightness of a stained site (mitochondria 41).
Fig. 7 is a diagram showing a configuration of a system of a Second Embodiment.
Fig. 8 is a fluorescence image obtained in an example.
Fig. 9 is a graph of brightness values measured in the example.

### BEST MODE FOR CARRYING OUT THE INVENTION

### [First Embodiment]

The following will describe a First Embodiment of the present invention. The present embodiment embodies a confocal fluorescence microscope system with the function of evaluating a cell function.
First, a configuration of the system is described.

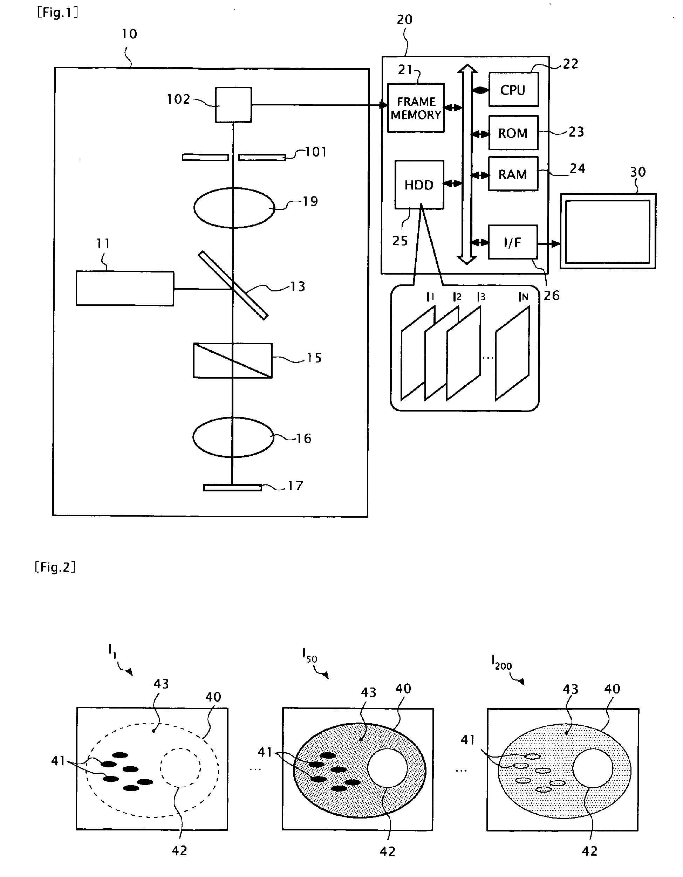

Fig. 1 is a diagram representing a configuration of the system. As shown in Fig. 1, the system includes a main body of microscope 10, a computer 20, and a monitor 30, among other components.

In the main body of microscope 10, a specimen 1 7 is disposed that includes living cells. The specimen 1 7 has been supplemented with a fluorescent dye for mitochondria (for example, RH123: Rhodamin 123). The fluorescent dye stains only the mitochondria in the living cells, leaving the other organelles unstained.
The main body of microscope 10 includes an excitation light source 11 that emits a laser beam. The laser beam includes at least a wavelength component that can serve as excitation light for the fluorescent dye (for example, a wavelength component of 507 nm). At least this wavelength component of the laser beam is reflected by a dichroic mirror 1 3 toward the specimen 1 7 through an optical scanner 15 and an objective lens 16, and forms a single light spot on the specimen 17. The fluorescent dye at this light spot generates fluorescence (529 nm), which is incident on the dichroic mirror 13 through the objective lens 16 and the optical scanner 15. The fluorescence travels through the dichroic mirror 13 and falls on a pinhole mask 101 through an image-forming lens 19.

The pinhole mask 101 is conjugate to the specimen 17, so that only the necessary light component of the fluorescence incident on the pinhole mask 101 passes through it. The fluorescence passing through the pinhole mask 101 enters a light sensor 102 where photoelectric conversion occurs. The fluorescence converted to an electrical signal in the light sensor 102 is then sent to the computer 20. In the computer 20, the electrical signal is converted to a digital signal and stored in a frame memory 21 of the computer 20.

In the main body of microscope 10, the optical scanner 15 and the light sensor 12 are driven in synchronism to two-dimensionally scan the specimen 1 7 with a light spot, thereby repeatedly generating electrical signals. As a result, a fluorescence image of one frame is obtained from the specimen 1 7 (imaging of the specimen 1 7). The imaging magnifications of the objective lens 16 and the image-forming lens 19 are set to values suitable for the observation of microstructures (organelles) of the living cells. Accordingly, the fluorescence image contains one to several living cells.

The main body of microscope 10 of the system repeats this imaging process N times, either continuously or intermittently, so that fluorescence images of N frames are obtained. For example, the imaging is repeated about 200 to 300 times (N ≈ 200 to 300), with the scan rate of the light spot and the power of the excitation light source 11 maintained constant in each imaging. During a non-imaging period of each frame, no light is incident on the specimen 17. Here, the number of imaging processes is proportional to the quantity of irradiated light on the specimen 17.

In each imaging, a CPU 22 in the computer 20 reads out digital signals accumulated in the frame memory 21 and creates a fluorescence image I of the specimen 17. The fluorescence image I is stored in a hard disc drive 25. After N times of imaging, fluorescence images I₁, I₂, ..., I_{N} of N frames are accumulated in the hard disc drive 25. As required, the fluorescence images I₁, I₂, ..., I_{N} are output to the monitor 30 via an interface circuit 26.

The computer 20 also includes a ROM 23 and a RAM 24, the former being a memory storing a basic operating program for the CPU 22, and the latter a memory used in the operation of the computer 20 when needed. The hard disc drive 25 also stores a system operating program for the CPU 22, which is read out at appropriate timings to cause the CPU 22 to perform various processes. In the system, the "evaluating processing of phototoxic property", described later, is included in these processes.

Fig. 2 shows an exemplary illustration of the fluorescence images I₁, I₂, ..., I_{N} obtained in the system as above. In Fig. 2, the shades are lighter as the brightness diminishes. The subscript "i" appended to the fluorescence image Iᵢ indicates a frame number, which is smaller for fluorescence images obtained earlier.
As shown on the left in Fig. 2, in the first fluorescence image I₁, mitochondria 41 at the stained site are the only bright component in a cell 40, and no other components, including a cell nucleus 42 and other cell organelles (dotted portion) at the non-stained site appear in the image. A cellular cytoplasm 43 is completely dark.

In the 50th fluorescence image I₅₀ shown in the middle in Fig. 2, the mitochondria 41 at the stained site appear darker as a result of bleaching, and the cellular cytoplasm 43 adjacent to the mitochondria 41 appears slightly brighter by fluorescing.
This phenomenon is the indication of the functional depression of the mitochondria 41 incurred by the irradiation of light in the imaging and the resulting extravasation of the fluorescent dye into the cellular cytoplasm 43 from the inner side of the mitochondrial membrane. It should be noted here that not all fluorescent dyes in the mitochondria 41 extravasate to the cellular cytoplasm 43. The extravasation stops at some time point.

In the 200th fluorescence image I₂₀₀ shown on the right in Fig. 2, the cellular cytoplasm 43 appears dark by bleaching, and stably maintains a small brightness value as does the mitochondria 41.
As described, fluorescence occurs not only in the mitochondria 41 at the stained site but in the cellular cytoplasm 43 at the adjacent site in the cell 40. This is related to the extravasation of the fluorescent dye from the mitochondria 41 to the cellular cytoplasm 43, i.e., functional depression of the mitochondria 41.

By taking advantage of this, the system sets a reference point on the cellular cytoplasm 43, and evaluates phototoxic property based on changes in brightness of the reference point.
In the following, description is made as to the evaluating processing of phototoxic property performed by the CPU 22. The process is performed after obtaining the fluorescence images I₁, ..., I_{N}.

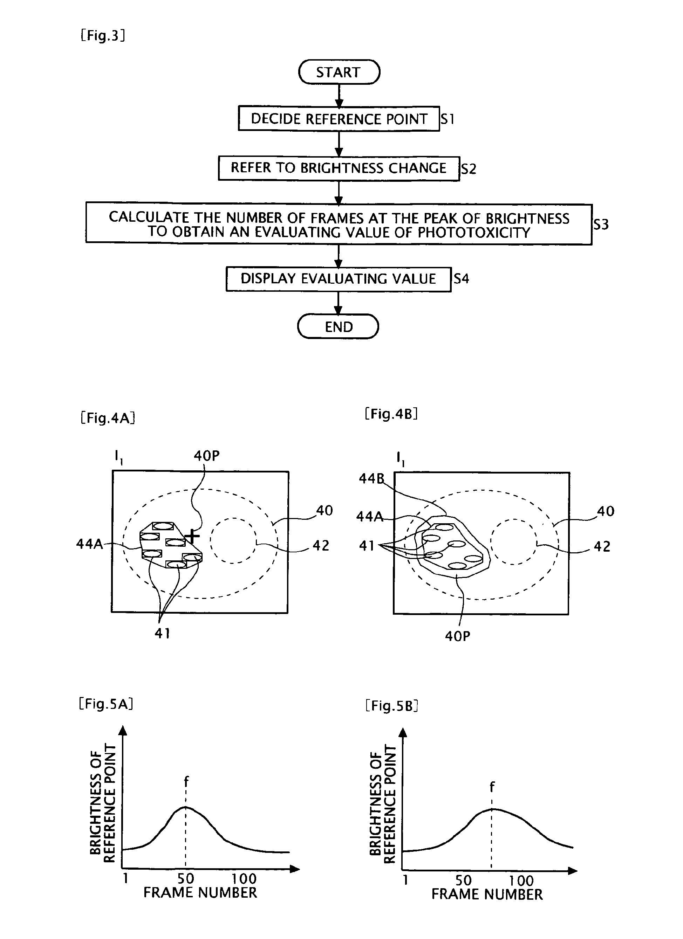

Fig. 3 is a flow chart representing the operation of the CPU 22 evaluating a phototoxic property. As shown in Fig. 3, the CPU 22 performs a process for determining a reference point (step S1), a process for referring to a brightness change of the reference point (step S2), a process for calculating an evaluating value (step S3), and a process for displaying the evaluating value (step S4), in this order from the top. The following describes each step in order.

### Step S1 (Process for Determining Reference Point)

In this step, as shown in Fig. 4(A), the CPU 22 refers to the first fluorescence image I₁ and compares the brightness value of each pixel of the fluorescence image I₁ with a threshold value to find pixels whose brightness values exceed the threshold value. The area where such pixels reside is regarded as a stained area 44A. Since the only fluorescing component in the first fluorescence image I₁ is the mitochondria 41, the area containing the mitochondria 41 is regarded as the stained area 44A.

The CPU 23 then sets a reference point 40P at coordinates separated from the stained area 44A by a small distance represented by predetermined coordinates. The predetermined coordinates have been set to appropriate values to locate the reference point 40P on the cellular cytoplasm 43.

### Step S2 (Process for Referring to Brightness Change of the Reference Point)

In this step, the CPU 22 extracts brightness values P₁, ..., P_{N} of the reference point 40P from the fluorescence images I₁, ..., I_{N}. The subscript "i" appended to the brightness value P indicates the frame number. These brightness values P₁, ..., P_{N} may come from a single pixel at the reference point 40P, or preferably from a plurality of pixels (pixels in an arbitrarily-shaped area) at the reference point 40P, in which case a mean brightness value or maximum brightness value of the pixels is used as the brightness value P₁, ..., P_{N}. Fig. 4(B) will be described later.

Fig. 5(A) shows a graph plotting the brightness values P₁, ..., P_{N} of the reference point 40P, in which the horizontal axis represents the frame number, and the vertical axis represents the brightness value. From these data, the CPU 22 finds changes in brightness of the reference point 40P.
As shown in Fig. 5(A), the brightness value of the reference point 40P increases as the frame number increases (increase in an amount of light irradiation), and reaches a peak in a certain frame (the 50th frame in the figure). The brightness value then decreases until it stabilizes at low brightness values in certain frames (around the 100th frame in the figure). The rate of increase of the brightness value indicates the chromogenic rate of the cellular cytoplasm 43, i.e., the functional depression rate of the mitochondria 41.

Here, when the frame number at which the brightness value has the peak is f, the frame number f becomes smaller as the functional depression rate of the mitochondria 41 becomes faster, and larger as the functional depression rate of the mitochondria 41 becomes slower. To test this, the functional depression rate was slowed by reducing the power of the excitation light source 11, with the other conditions held constant. As expected, the frame number f increased, as shown in Fig. 5(B).

### Step S3 (Process for Calculating Evaluating Value)

In this step, the CPU 22 calculates a frame number f (50 in Fig. 5(A)) at which the brightness value of the reference point 40P has the peak, as shown by the arrow in Fig. 5(A). Based on this frame number f, the CPU 22 calculates an evaluating value E of phototoxic property. The evaluating value E is given by, for example, E = 1 /f, E = α/f, E = f_{A} - f, or E = f_{A} - αf (where α and f_{A} are constants), so that larger evaluating values E are obtained as the functional depression rate of the mitochondria 41 becomes faster.

### Step S4 (Process for Displaying Evaluating Value)

In this step, the CPU 22 displays the calculated evaluating value E on the monitor 30. Here, it is preferable that the CPU 22 display the current fluorescence image I_{N} along with the evaluating value E, and, as a marker for an operator, a mark such as a crosshair cursor or a rectangular shaped frame superimposed on the reference point 40P.

As described, the system evaluates phototoxic property through repeated imaging of the specimen 1 7 performed by the main body of microscope 10. The evaluation is performed based on brightness changes (Fig. 5) at the adjacent site (here, the cellular cytoplasm 43) of the stained site (here, the mitochondria 41), which is referred to instead of the brightness changes at the stained site. This evaluation yields proper results because the brightness changes at the adjacent site (here, the cellular cytoplasm 43) are well correlated with the functional depression at the stained site (here, the mitochondria 41), as described above.

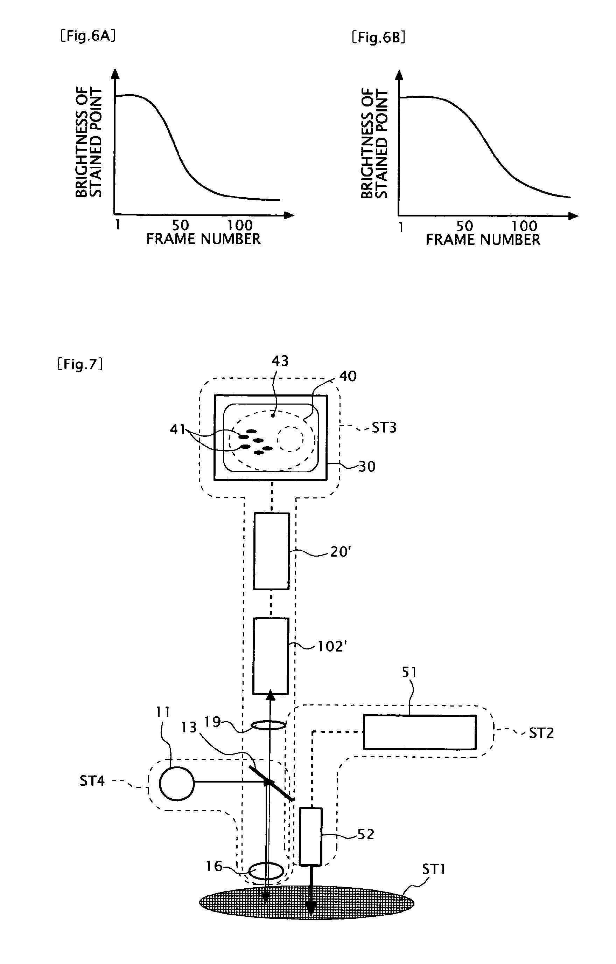

Further, in the evaluation performed by the system, because the frame number f at which the brightness value at the adjacent site (here, the cellular cytoplasm 43) has the peak is reflected in the evaluating value E, the evaluating value E accurately reflects the chromogenic rate of the adjacent site (here, the cellular cytoplasm 43) or the functional depression rate of the stained site (here, the mitochondria 41). That is, the evaluating value E is an accurate indication of phototoxic property.
For comparison, Fig. 6(A) and Fig. 6(B) show brightness changes at the stained site (here, the mitochondria 41). Fig. 6(A) represents a graph obtained with the excitation light source 11 held at high power, and Fig. 6(B) represents a graph obtained with the excitation light source 11 held at low power. As shown in Fig. 6(A) and Fig. 6(B), the brightness change curves obtained from the stained site (here, the mitochondria 41) at different power levels of the excitation light source 11 have the peaks at the same point (frame number 1). It is therefore difficult to calculate an evaluating value of phototoxic property from these brightness change curves.

Further, as described, while the brightness change curve from the stained site (here, the mitochondria 41) shows there is bleaching at the stained site (here, the mitochondria 41), it does not necessarily mean there is functional depression. As such, the evaluating value of phototoxic property calculated from this brightness change curve would not be as accurate as the evaluating value E obtained in this embodiment.

### (Others)

In this system, it is preferable that the mathematical formula deriving the evaluating value E from the frame number f be appropriately formulated such that the actual phototoxic property and the evaluating value E are linearly related to each other. This is possible by experiments or simulations using systems including the living cell in which the phototoxic property is known.

Further, in this system, the evaluating value E of phototoxic property is defined by the frame number f at the peak brightness value. However, the evaluating value E may be defined by the brightness value (peak brightness value) when the brightness has the peak. Further, the evaluating value E may also be defined by both the frame number f and the peak brightness value.
The foregoing description of the present embodiment was given through the case where fluorescence images of about 200 frames were obtained, in order to illustrate the evaluation of phototoxic property in the stained area 44A using changes in brightness value of the reference point 40P. However, from the standpoint of preventing unnecessary damage to the cells, it is preferable that the irradiation of a laser beam from the excitation light source 11 be stopped or the intensity of the laser beam be reduced immediately after the brightness value of the reference point 40P has reached the peak and starts to decline, or after a predetermined period of time (several seconds) has elapsed from such an event.

Because the system uses a confocal microscope as the main body of microscope 10, a plurality of fluorescence images with different sectionings can be obtained. Such multiple fluorescence images can be used to improve the accuracy of evaluating value E.
The present embodiment has been described through the case where the stained site is mitochondria 41; however, other organelles or the area outside of the cell membrane (culture fluid) may be used as the stained site. When the stained site is the cell nucleus, the reference point may be set on the cellular cytoplasm 43. When the stained site is the cellular cytoplasm 43, the reference point may be set on the culture fluid. Further, when the stained site is the culture fluid, the reference point may be set on the cellular cytoplasm 43. In any case, the reference point is set at a site adjacent to the stained site with a membrane in between.

In the foregoing description, the computer 20 performs each process of the system. However, the operation of the computer 20 may be executed either partially or entirely by a device (control device, image processing device) designated to the main body of microscope 10, or by an operator.
For example, the reference point 40P, which was described as being automatically decided by the computer 20 in the system, may be entered by an operator through an input device (keyboard, mouse, or the like; not shown). When an operator is allowed to enter the reference point 40P, a marker may be superimposed on the fluorescence image I1 displayed on the monitor 30 as shown in Fig. 4(A) and Fig. 4(B). The marker may be a crosshair cursor (Fig. 4(A)) or a rectangular shaped frame. Alternatively, the marker may be an arbitrarily-shaped frame, such as a closed and free curved frame or a closed and multiangular shaped frame, that is displayed to exclude the stained area 44A (mitochondria 41) from the area including the stained area 44A and the cellular cytoplasm 43 adjacent thereto (Fig. 4(B)). In the example shown in Fig. 4(B), the area defined by the annular frame is the reference point 40P. The arbitrarily-shaped frame defining the reference point 40P may be variable in size.

The main body of microscope 10 of the system, which has been described as a microscope that obtains fluorescence images, may be modified to obtain both fluorescence images and differential interference images. In this case, the differential interference image may be superimposed on the fluorescence image displayed on the monitor 30. The superimposed differential interference image allows for observation of non-fluorescing organelles (transparent organelles). Further, the differential interference image can be used to set the reference point. In this case, failure to set the reference point becomes less likely.

Further, in this system, the operating program for the CPU 22 has been described as being pre-stored in the hard disc drive 25. However, the program may be installed either partially or entirely in the computer 20 via, for example, the Internet or CD-ROM (not shown).
Further, the main body of microscope 10 of the system, described as a confocal microscope that detects a confocal point of the light from the specimen 17, may omit this function. In this case, the pinhole mask 101 will not be required. Further, the main body of microscope 10 may be modified to a multiphoton microscope that attains the confocal effect by methods other than using the pinhole mask.

Further, the main body of microscope 10 of the system, which is a scanning microscope for scanning the specimen 1 7 with light, may be a non-scanning microscope when it omits the confocal point detecting function. In this case, the optical scanner 15 will not be required, and an imaging sensor is provided instead of the light sensor 102.
Further, the system is applicable to evaluation of cell function using a drug, by supplying a drug to the mitochondria 41 with the fluorescent dye. Further, the system is applicable to evaluation of cell function by heat or radiation, by applying heat or radiation with light.

### [Second Embodiment]

The following will describe a Second Embodiment of the present invention. The present embodiment embodies a photodynamic therapy system, and a photodynamic therapy method using it.
Fig. 7 is a diagram showing a configuration of the present system. As shown in Fig. 7, the system is primarily made up of four components, including a therapeutic objective ST1, a therapeutic system ST2, an observing system ST3, and an excitation system ST4.

The therapeutic objective ST1 is, for example, an affected area including cancer cells, supplemented beforehand with a fluorescent dye for mitochondria (for example, RH123). The fluorescent dye is used for the evaluation of phototoxic property (evaluation of therapeutic effect).
The therapeutic system ST2 irradiates the therapeutic objective ST1 with radiation rays (such as gamma rays) or laser light for therapy (ultraviolet range, visible range, infrared range), so as to induce cell injury or cell death in cancer cells. The gamma rays have the effect of solely inducing cell injury, while the laser light for therapy induces cell injury (or cell death) by reacting with the fluorescent dye applied to the therapeutic objective ST1. The following describes the case using the latter (PDT).

The laser light for therapy is generated in a radiation device 51 provided in the therapeutic system ST2, and is emitted as pulsed oscillations toward the therapeutic objective ST1, from a tube tip (head) 52, measuring several millimeters to several centimeters in diameter, provided at the tip of the therapeutic system ST2. The head 52 is provided to improve the efficiency of concentrating the energy of the laser light for therapy onto the therapeutic objective ST1.
The excitation system ST4 includes an excitation light source 11 and a dichroic mirror 13. Through an objective lens 16 of the observing system ST3, the excitation system ST4 emits excitation light (for example, a wavelength of 507 nm) as pulsed oscillations toward the therapeutic objective ST1. The excitation light is emitted alternately with the laser light for therapy.

The observing system ST3 includes the objective lens 16, an image-forming lens 19, an imaging sensor 102', a circuit part 20', and a monitor 30, among others. The fluorescence generated in the therapeutic objective ST1 during the irradiation of the excitation light is captured by the objective lens 16 and the image-forming lens 19 of the observing system ST3, and a fluorescence image of the therapeutic objective ST1 is formed on the imaging sensor 102'. The imaging sensor 102' continuously captures the fluorescence images, which are then output to the monitor 30 one after another via the circuit part 20'.

The imaging magnifications of the objective lens 16 and the image-forming lens 19 of the observing system ST3 are set to values suitable for the observation of microstructures (organelles) of the cells. Accordingly, cells 40 of the therapeutic objective ST1 are displayed in real time on the monitor 30.
It should be noted here that the head of the observing system ST3 and the excitation system ST4, and the head 52 of the therapeutic system ST2 are facing substantially the same point on the therapeutic objective ST1, so that the imaging point of the fluorescence image substantially coincides with the irradiation point of the laser light for therapy. To suppress any misregistration between the two, these heads may be fixed or the same head may be used.

During a course of therapy, an operator observes the stained site (here, the mitochondria 41) and the adjacent site (here, the cellular cytoplasm 43) on the monitor 30 while the therapeutic objective ST1 is being irradiated with the laser light for therapy. Here, the operator looks at the brightness of the adjacent site (here, the cellular cytoplasm 43) and evaluates the phototoxic property (therapeutic effect) of the therapeutic system ST2 according to the timing at which the brightness reaches the peak, or the extent of brightness when it has the peak. According to the result of evaluation, the operator suspends the irradiation of the laser light for therapy at an appropriate timing, or adjusts the power of the therapeutic system ST2 at an appropriate level.

In this manner, the system allows the operator to perform photodynamic therapy while evaluating the therapeutic effect in real time, making it possible to perform an appropriate therapy without failing to remove cancer tissues by underexposure of the laser light for therapy, or without causing any side effect by overexposure of the laser light for therapy.
While the system was described in which the operator visually checks the brightness of the adjacent site (here, the cellular cytoplasm 43), the brightness may be checked by automation. In this case, the circuit part 20' of the observing system ST3 extracts a brightness signal of the adjacent site (here, the cellular cytoplasm 43) from the output of the imaging sensor 102', and notifies the operator of the level of the brightness signal in real time. The notification may be given on the monitor 30, or by playing sounds from a separately provided sound output device (speaker).

Further, while the excitation light and the light for therapy are separately provided in the system described above, the light for therapy may be used to also provide the excitation light, when it contains a wavelength component for the excitation light.
The therapeutic apparatus described in this Second Embodiment can be made into a diagnostic apparatus simply by replacing the therapeutic system ST2 with a diagnostic system. The diagnostic system includes a diagnostic wave (sound wave, electromagnetic wave) generator, an illuminating optical system for illuminating the affected area, an imaging sensor, and an imaging optical system for condensing the reflected light from the illuminated affected area onto the imaging sensor.

### [Example]

The following describes an example of the evaluation of cell function according to the present invention, performed with the confocal fluorescence microscope system of the First Embodiment.
RH123, used as a fluorescent dye, was applied to the mitochondria in living cells to prepare a specimen. The specimen was two-dimensionally scanned by irradiating an argon laser (488 nm) emitted in a predetermined intensity from the excitation light source 11, so as to obtain a fluorescence image of one frame.

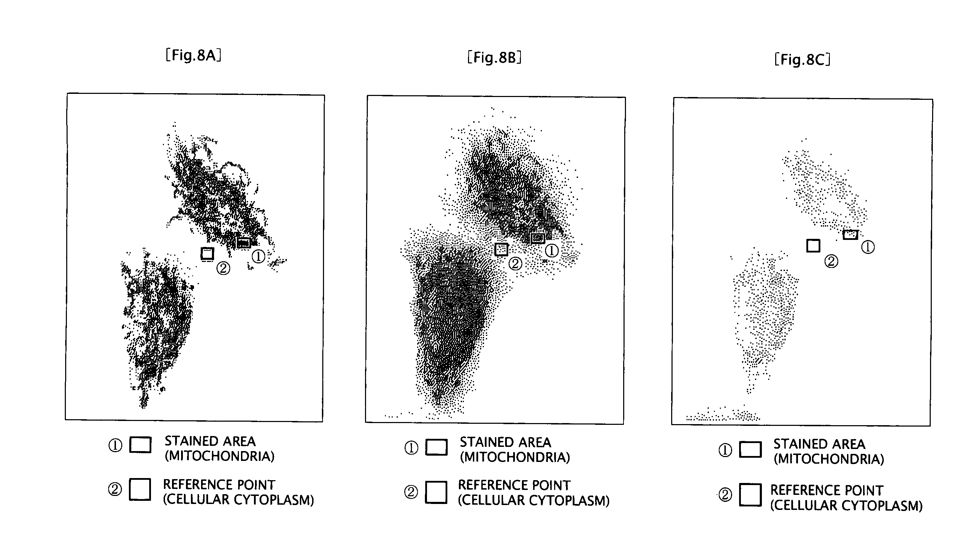

Fig. 8(A) is the actual first fluorescence image obtained. Predetermined positions of the mitochondria (stained area) and the cellular cytoplasm (reference point) were specified by rectangular shaped frames, and a mean value of brightness values of the pixels in each of these specific areas was determined. These mean values were used as the brightness value of the mitochondria, and the brightness value of the cellular cytoplasm, respectively.
This procedure of obtaining the fluorescence image was repeated under the same conditions, and the brightness values of the mitochondria and the cellular cytoplasm were measured. Fig. 8(B) is the actual 50th fluorescence image, and Fig. 8(C) is the actual 150th fluorescence image obtained. It can be seen from Fig. 8 that the fluorescence image indeed undergoes changes as described in the First Embodiment with reference to Fig. 2.

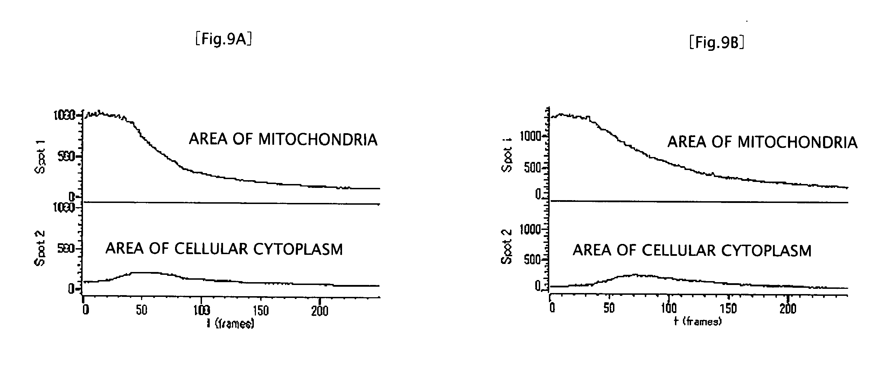

Fig. 9(A) is the actual graph plotting the brightness values of the mitochondria and the cellular cytoplasm measured by the irradiation of an argon laser of a predetermined intensity. In Fig. 9(A), the decrease in the brightness value of the mitochondria is due to bleaching and a release of RH123 into the cellular cytoplasm. The increase of the brightness value of the cellular cytoplasm is due to the RH123 released by the mitochondria, and the peak indicates the end of the RH123 release into the cellular cytoplasm. That is, the graph tells that the functional depression of the mitochondria is complete when the image with the frame number 50 is obtained, in which the brightness value of the cellular cytoplasm has the peak.

The brightness values of the mitochondria and the cellular cytoplasm were also measured under the same conditions except for reducing the intensity of the laser beam emitted by the excitation light source 11. Fig. 9(B) is the actual graph plotting the brightness values of the mitochondria and the cellular cytoplasm measured by the irradiation of a laser beam having a weaker intensity than the laser used in Fig. 9(A). As can be seen in Fig. 9(B), the brightness value of the cellular cytoplasm has the peak in frame number 70, showing that the functional depression of the mitochondria proceeds at a slower rate when the intensity of the excitation laser beam is weaker.

### INDUSTRIAL APPLICABILITY

An evaluating method of the present invention is applicable to evaluation of an illuminant such as a laser scanning confocal microscope (evaluation of device effectiveness), evaluation of drugs such as antioxidants (drug screening, etc.), and evaluation of fluorescent dye (evaluation of fluorescence lifetime), among others.

## Claims

1. An evaluating method of cell function concerning a living cell, said method comprising:
dyeing operation of dyeing a specific site of said living cell with a fluorescent dye;
measuring operation of measuring a brightness value of fluorescence generated at an adjacent site of said specific site as a result of irradiation of said stained living cell with light; and
evaluating operation of evaluating said cell function based on changes in said measured brightness value.

2. The evaluating method of cell function according to claim 1, wherein said fluorescent dye is Rhodamin 123.

3. The evaluating method of cell function according to claim 1 or 2, further comprising the operation of controlling the irradiation of light to said stained living cell at a predetermined timing according to said evaluation of cell function.

4. The evaluating method of cell function according to any one of claims 1 through 3, wherein said cell function is evaluated based on a peak of a curve representing said brightness changes in said evaluating operation.

5. The evaluating method of cell function according to claim 4, wherein said cell function is evaluated based on an amount of light irradiation being spent until said brightness change curve reaches the peak in said evaluating operation.

6. The evaluating method of cell function according to claim 4, wherein said cell function is evaluated based on a brightness value at the peak of said brightness change curve, in said evaluating operation.

7. The evaluating method of cell function according to any one of claims 1 through 3, wherein the adjacent site of said specific site is one of an area specified by a rectangular shaped frame, and an area specified by one of a closed and free curved frame, and a closed and multiangular shaped frame in which said specific site is excluded from an area including the specific site and an area adjacent to the specific site.

8. The evaluating method of cell function according to claim 7, wherein said brightness value is one of a maximum brightness value and a mean brightness value of a plurality of fluorescence brightness values measured in said adjacent site.

9. The evaluating method of cell function according to any one of claims 1 through 8, wherein said specific site is a mitochondrion, and wherein said adjacent site is a cellular cytoplasm.

10. An evaluating system of cell function that evaluates a cell function concerning a living cell,
said living cell including a specific site stained with a fluorescent dye in advance,
said evaluating system of cell function comprising:
an irradiating unit that irradiates said stained living cell with light;
a measuring unit that measures a brightness value of fluorescence generated at an adjacent site of said specific site as a result of said irradiation of light; and
an evaluating unit that evaluates said cell function based on changes in said measured brightness value.

11. The evaluating system of cell function according to claim 10, wherein said fluorescent dye is Rhodamin 123.

12. The evaluating system of cell function according to claim 10 or 11, further comprising a controlling unit that controls the irradiation of light to said stained living cell at a predetermined timing according to said evaluation of cell function.

13. The evaluating system of cell function according to any one of claims 10 through 12, wherein said evaluating unit evaluates said cell function based on a peak of a curve representing said brightness changes.

14. The evaluating system of cell function according to claim 13, wherein said evaluating unit evaluates said cell function based on an amount of light irradiation being spent until said brightness change curve reaches the peak.

15. The evaluating system of cell function according to claim 13, wherein said evaluating unit evaluates said cell function based on a brightness value at the peak of said brightness change curve.

16. The evaluating system of cell function according to any one of claims 10 through 12, wherein the adjacent site of said specific site is an area specified by a rectangular shaped frame, or an area specified by a closed and free curved frame or a closed and multangular shaped frame in which said specific site is excluded from an area including the specific site and an area adjacent to the specific site.

17. The evaluating system of cell function according to claim 16, wherein said brightness value is one of a maximum brightness value and a mean brightness value of a plurality of fluorescence brightness values measured in said adjacent site.

18. A fluorescent microscope system, comprising:
an excitation unit that irradiates a living cell with excitation light;
an observing unit that acquires a fluorescence image of said living cell; and
the evaluating system of cell function of any one of claims 10 through 17,
wherein said excitation unit and said observing unit are also used as said irradiating unit and said measuring unit, respectively, of said evaluating system.

19. A photodynamic therapy method that irradiates a living cell with light, said method comprising:
dyeing operation of dyeing a specific site of said living cell with a fluorescent dye;
therapeutic operation of irradiating said stained living cell with light; and
evaluating operation of evaluating a cell function concerning said living cell by the evaluating method of cell function of any one of claims 1 through 9.

20. A photodynamic therapy system that irradiates a living cell with light, said living cell including a specific site stained with a fluorescent dye in advance,
said system comprising:
a therapeutic unit that irradiates said stained living cell with light;
a measuring unit that measures a brightness value of fluorescence generated at an adjacent site of said specific site as a result of said irradiation of light; and
a presenting unit that presents to an operator changes in said measured brightness value, in real time.

21. The photodynamic therapy system according to claim 20, wherein said fluorescent dye is Rhodamin 123.

22. The photodynamic therapy system according to claim 20 or 21 , wherein the adjacent site of said specific site is one of an area specified by a rectangular shaped frame, and an area specified by one of a closed and free curved frame, and a closed and multiangular shaped frame in which said specific site is excluded from an area including the specific site and an area adjacent to the specific site.

23. The photodynamic therapy system according to claim 22, wherein said brightness value is a maximum brightness value or a mean brightness value of a plurality of fluorescence brightness values measured in said adjacent site.
